# EUROPEAN PATENT APPLICATION

(11) **EP 2 020 447 A1**
(43) Date of publication of application: **04.02.2009**
(21) Application number: 07380229.0
(22) Date of filing: 03.08.2007
(51) Int. Cl.: C12Q 1/68, G01N 33/50

(54) **Method for identifying compounds modulating adiponectin gene expression**

(71) Applicant: Zeltia, S.A., 28003 Madrid (ES)
(72) Inventor: Santamaría Núñez, Gema, 28002 Madrid (ES)
(74) Representative: ABG Patentes, S.L.

(57) **Abstract**

The invention relates to a method for identifying suitable compounds for the treatment and/or prevention of metabolic diseases in which there are altered adiponectin levels, which method uses reporter cell systems containing DNA constructs comprising the adiponectin gene promoter region operatively coupled to a reporter gene and in which the compounds causing an alteration in the reporter gene expression form candidates that are useful in the treatment or prevention of said metabolic diseases.

SEQUENCE LISTING

<110> ZELTIA S.A.

<120> METHOD FOR IDENTIFYING COMPOUNDS MODULATING METABOLIC DISEASE-
RELATED GENE EXPRESSION

<130> P3159EP00

<160> 3

<170> PatentIn version 3.4

<210> 1
<211> 2274
<212> DNA
<213> Homo sapiens

<400> 1
tagtggagac ggggtttcac catcttcgtc aggctggtct cagactcctg acctcaagtg 60

atctgcccgc ctcagcctcc caaaatgctg ggattacagg catgagctac tgtgcccggc 120

caaagaaaat actttttatg ccagccctga aactaccctg aagcacatac atcaaccttg 180

aggcctcaca ctccatcaag aggggtgaag ggcatgagga attagaaagc atagggattt 240

ttagttagac agatctggtt caaatcctag acttgtgcct tgaacaaatt atttaccctc 300

attgaactct agattcatta tttgtaaaat gaaagacaat aatagttatc tccaaaggaa 360

agttgaatat gatcattcat ttattcatta attcaacatt tattattgcc tactttgtgc 420

caggttctat tctaggaact aagggataca actttgaata ggcaaaatct ctgctctcct 480

gaagtttact tttttttttt tttttgagac agagtttcac tcttgtcacc caggctagag 540

cgcaatggtg ctcttggctc actgcaacct ccacctcctg ggttcaagtg attctcttgt 600

ctcagcctcc caagtagctg ggactacagg tatgtgccac cacgcccggc tatttctgca 660

tttttagtag agatggggtt tcaccatgtt ggccagactg gtctcaaact cctgatctca 720

ggtgatatgc ctgtcttggc cttccaaagt actgggatta caggcctgag ccactgcacc 780

tgacctgaag tttatgttct attaaatagc aacagacagt aacataaacc aaaaataaat 840

aggaaaacac cataacaaaa atcaaacagt gatataattg agagttgctt ctatttcttt 900

ttgttgtctt cttggttcaa tcagcctgct aaactatatg gaacctcatt ttcatgggcc 960

acttatttaa gccgggggac cttggaaagt ctctcatgtc tctcatctca acggcctaat 1020

gtgacttctc ttgaaatatt tggacattag caggaagctg aggctttaca tcagatcttt 1080

actttaatgg tggacttgac tttactggta gatttttagg ctctgtgtgg actgtggaga 1140

tgatatctgg ggggcaggca gacacttgcc ctgcctctgt ctgagaaaat tctgttttgg 1200

atgtcttgtt gaagttggtg ctggcatcct aagcccttgc tggggtcgta atttaattca 1260

tcagaatgtg tggcttgcaa gaaccggctc agatcctgcc cttcaaaaac aaaacatgag 1320

cgtgccaaga aagtccaagg tgttgaatgt tgccacttca agcctaaact ttctaggaac 1380

acctaagtgg gtggcagctt ccagttctcc aggctgcttc taggccagag ctgggttcca 1440

caagagacag aataggcata tatatgctta aggaactgga aaaacaggct ctctctctct 1500

cacaaacaca cacacacaca taccaaggta gctgtcaaaa tgttatccga aattttggaa 1560

ccaaaaaatc ttgaaagatg gtattccaat atcacatttt atgtaagttt tctattatat 1620

tagattcaaa ttacgattcg aggccacaag ctttaagaat tcagggcctt tttaacttgc 1680

caagccccac accactccag gaacttcccc acaccccagt tctcagaatt catgtgcaag 1740

gtctttccta aatccagggt ccaggtcaga gagtggagga tgtgctctat ttcttacctg 1800

attgcagacc cctctgacag tgctcccttc tgaagcactc actgtctgaa cgtacacagt 1860

ctcagactta atcatgcaca gtgagcaaga ctgtggtgtg ataattggcg tccctgactt 1920

attagggcaa atctatggga gggggagacc tcctggacca ctgagcaatt aattcattta 1980

cattaggaag tttctccgtc agatgcagga aaaaaatctt gttttcctgc tgtggttttg 2040

acttttgccc catcttctgt tgctgttgta ggaggcaaaa taagggtcaa ggcctggaaa 2100

cacaagtgct ttgactgaag ctccacttgg cttccgaagc ccaagctggg ttgtaccagg 2160

ttccctaggg tgcaggctgt gggcaactgc cagggacatg tgcctgccca ccggcctctg 2220

gccctcactg agttggccaa tgggaaatga caattgtgag gtggggactg cctg 2274

<210> 2
<211> 30
<212> DNA
<213> Artificial sequence

<220>
<223> Forward primer for amplifying the human adiponectin gene promoter
      region

<400> 2
tataacgcgt tagtggagac ggggtttcac 30

<210> 3
<211> 25
<212> DNA
<213> Artificial sequence

<220>
<223> Reverse primer for amplifying the human adiponectin gene promoter
      region

<400> 3
ctcgagcagg cagtccccac ctcac 25

## Description

### TECHNICAL FIELD OF THE INVENTION

The present invention relates to a method for identifying compounds which can increase the expression of genes involved in different metabolic diseases, such as diabetes, obesity, metabolic syndrome, arteriosclerotic cardiovascular disease, dyslipidemia or lipodystrophy.

### BACKGROUND OF THE INVENTION

Insulin resistance is a pathology characterized by the inability of normal circulating insulin levels to cause a normal response in insulin-sensitive tissue (muscle, adipose tissue and liver). Insulin resistance in adipose tissue results in an increase of triglyceride hydrolysis, insulin resistance in muscles results in a decrease in glucose uptake by the liver and insulin resistance in the liver results in a decrease of intracellular glucose reserves, the end result of which is an increase of blood sugar, which finally causes type II diabetes and metabolic syndrome.

### Adiponectin

Adipose tissue is an important endocrine organ, secreting multiple metabolically active important proteins referred to as adipokines. Some known adipokines are leptin, tumor necrosis factor (TNF)-α, interleukin (IL)-6, adipsin and adiponectin. Most adipokines are secreted by adipocytes *per se,* although some can be secreted by other cell types in adipose tissue.

Adiponectin is a protein synthesized by white adipose tissue, circulating at relatively high plasma concentrations (2-30 µg/ml), it has an important role in glucose and lipid metabolism. Several studies have demonstrated the relationship between adiponectin and insulin sensitivity. A relationship between adiponectin and cardiovascular diseases has also been proven.

Adiponectin is a 247-amino acid protein (Mr 30 kDa) consisting of 4 domains. The first domain is a signal peptide located in the amino-terminal area allowing the secretion of the hormone outside the adipocytes; the second domain is a 28-amino acid region varying among species, the third domain is a collagenous domain formed by 22 glycine-X-tyrosine triplets; and finally a globular domain in the carboxy-terminal region.

Adiponectin molecules undergo a post-translational modification within the adipocyte, being grouped in multimeric forms, including trimers, hexamers and oligomers with a high molecular weight. Monomeric adiponectin is not usually detected in the blood flow.

Low plasma adiponectin levels are associated to adverse metabolic conditions, such as diabetes, metabolic syndrome, dyslipidemia, lipodystrophy and arteriosclerotic cardiovascular disease. It has been found that in these disorders there is also reduced adiponectin mRNA expression in plasma and adipose tissue; it has been considered that this can be due to an altered adipocyte functioning.

### Adiponectin and Obesity

Unlike other adipokines, the concentration of which increases as the lipid mass increases, the circulating adiponectin levels are paradoxically reduced in obese individuals, the circulating adiponectin levels being inversely proportional to the body mass index (BMI) and to the body fat percentage (Arita Y et al., Biochem Biophys Res Commun 1999; 257 (1): 79-83; Cnop M et al., Diabetologia 2003; 46 (4) : 459-469*;* Kern PA et al . , Diabetes 2003; 52 (7): 1779-1785)*.*

### Adiponectin and Insulin Sensitivity

Insulin resistance is a pathology characterized by the inability of normal circulating insulin levels of producing a normal response in insulin-sensitive tissue (muscle, adipose tissue and liver). Insulin resistance in adipose tissue results in an increase of triglyceride hydrolysis, insulin resistance in muscles results in a decrease of the glucose uptake by the liver and insulin resistance in the liver results in a decrease of intracellular glucose reserves, the end result of which is an increase of glycemia, which finally results in type II diabetes and metabolic syndrome. Several studies have demonstrated a correlation between plasma adiponectin levels and insulin sensitivity (Berg AH et al., Nat Med 2001; 7 (8): 947-953; Combs TP et al., Endocrinology 2002; 143 (3): 998-1007*;* Hotta K et al., Diabetes 2001; 50 (5): 1126-1133*;* Steffes MW et al., Ann Epidemiol 2004; 14 (7): 492-498 and Weyer C et al., J Clin Endocrinol Metab 2001; 86 (5): 1930-1935*).* In fact, a reduction in adiponectin levels could identify a resistance to insulin before the development of diabetes *per se,* according to some studies (for example, Hotta K et al *supra*.). Other studies have associated high adiponectin levels with a reduced risk of developing type two diabetes in multiple ethnic groups (Daimon M et al., Diabetes Care 2003; 26 (7): 2015-2020*;* Lindsay RS et al., Lancet 2002; 360 (9326): 57-58*;* Spranger J et al., Lancet 2003; 361 (9353): 226-228*).*

### Adiponectin and Cardiovascular Disease

Reduced adiponectin levels are also associated to coronary heart disease (Hotta K et al. Arterioscler Thromb Vasc Biol 2000; 20 (6) : 1595-1599*;* Pischon T et al., JAMA 2004; 291 (14): 1730-1737*).* The second study has shown that subjects with high adiponectin levels had a significantly reduced risk of myocardial infarction. Several studies have studied the relationship between adiponectin and cardiovascular risk factors (Hotta K et al., Arterioscler Thromb Vasc Biol 2000; 20 (6): 1595-1599*;* Shetty GK et al, Diabetes Care 2004; 27 (10): 2450-2457; Mantzoros CS et al, J Clin Endocrinol Metab 2005; 90 (8): 4542-4548*;* Schulze MB et al., Diabetes Care 2004; 27 (7): 1680-1687). These results suggest that adiponectin has an important role in atherosclerotic cardiovascular disease.

### Therapeutic Effects of Adiponectin

The administration of adiponectin to murine diabetes and lipoatrophy models has shown an improvement of insulin sensitivity. Both in wild-type mice and murine type 1 and type 2 diabetes models, the peritoneal injection of adiponectin caused a significant reduction of glucose levels (Berg AH et al., Nat Med 2001, 7 (8): 947-953;). The same effect has been obtained by means of the systemic infusion of the adiponectin globular domain to animal obesity, diabetes and lipoatrophy models (Yamauchi T et al., Nat Med 2001, 7 (8): 941-946*).* International patent application WO/2007/008937 describes adiponectin variants characterized by having a greater solubility, greater expression, stability and improved pharmacokinetic and pharmacodynamic parameters compared to adiponectin.

The effects of adiponectin overexpression have also been studied in animal models. Transgenic mice overexpressing globular adiponectin showed protection against resistance to insulin induced by a high-fat diet (Yamauchi T et al., J Biol Chem 2003, 278 (4): 2461-2468). Other transgenic mice, with chronically high adiponectin levels, showed an increase in lipid clearance, as well as in the suppression of insulin -mediated endogenous glucose production (Combs TP et al., Endocrinology 2004, 145 (1): 367-383). In another murine atherosclerosis model, it was shown that globular adiponectin can protect against atherosclerosis (Yamauchi T et al.,J Biol Chem 2003, 278 (4): 2461-2468).

### The activation of the expression of metabolism-related genes as therapeutic target

In spite of the benefits shown in the direct administration of adiponectin to animal models, it would be complicated to administer adiponectin directly to human subjects due to the large protein structure of adiponectin, as well as due to need for its post-translational processing. Therefore, there is a need of achieving activation of adiponectin expression in the organism by means of administering compounds activating adiponectin expression. As an example of such compounds, several studies have shown that treatment with thiazolidinediones causes an increase in circulating adiponectin levels in human subjects. This has been observed with rosiglitazone (Combs TP et al., Endocrinology, 2002 143: 998-1007; Tiikkainen M et al., Diabetes 2004, 53: 2169-2176) as well as with pioglitazone (Hirose H et al., Metabolism 2002, 51: 314-317) and with troglitazone (Phillips SA et al., Diabetes 2003, 52 (3): 667-674; Maeda N et al.,Diabetes 2001, 50: 2094-2099; Yu JG et al., Diabetes 2002, 51: 2968-2974).

The identification of compounds which can activate adiponectin expression requires having highly sensitive screening methods which are simple enough to enable screening a high number of compounds simultaneously. Screening methods including the two indicated features (sensitivity and simplicity) include the methods using cell systems in which the promoter region of the gene being studied is operatively coupled to a reporter gene, for which there are highly sensitive detection techniques. The compounds causing an increase in reporter gene expression will thus be those causing an increase in the transcription of the gene being studied. It is necessary to have the gene promoter region the activation of which is to be studied in order to carry out this type of assays. The adiponectin gene promoter has been described by Takahashi, M. et al. (Int. Journal of Obesity, 24:861-868). In addition, Kita, Al. et al (Biochem.Biophys.Res.Commun, 2005, 331:484-490) have shown the possibility of using said promoter to regulate the expression of reporter genes operatively coupled to the latter. To that end, they have used transient chimeric gene expression cell systems comprising the adiponectin gene promoter region fused to a reporter gene (luciferase), in which the reporter gene expression increases by means of the simultaneous cotransfection of the transcription factor C/EBP-beta and is negatively regulated after adding TNF-α to the cell culture. However, this document does not contemplate the possibility of using said cellular system for identifying additional compounds which can modulate adiponectin expression.

European patent application EP1577388 describes methods for identifying compounds which can activate adiponectin gene expression by means of using cells transiently expressing a chimeric gene comprising the adiponectin promoter region in which the PPARγ and RXRα dimer binding site and a reporter gene (luciferase) are located. However, the baseline reporter gene expression is very low, which requires the cotransfection of the PPARγ and RXRα dimers.

It is therefore necessary to have additional methods for screening compounds which can increase adiponectin expression and the expression of other genes acting in the same route in which the drawbacks of the methods known in the art do not exist.

### SUMMARY OF THE INVENTION

In a first aspect, the invention relates to a method for identifying suitable compounds for the treatment or the prevention of a metabolic disease presenting altered adiponectin levels or in which it is necessary to alter the adiponectin levels, comprising the steps of
(a)putting a cell in contact with a candidate compound in any degree of purity wherein said cell stably expresses a DNA construct comprising
   (i) the adiponectin gene promoter region or a functionally equivalent variant thereof and
   (ii) a reporter gene operatively coupled to said promoter region
   and
(b)detecting the reporter gene expression
wherein the assayed compound is identified as suitable for the treatment or prevention of said metabolic disease if it causes a variation in the reporter gene expression in an opposite direction to the existing alteration in the adiponectin levels in the metabolic disease compared to the alteration of the expression in the absence of said compound, or if it causes an alteration in reporter gene expression in the same direction as the alteration to be achieved in the adiponectin levels.

In a second aspect, the invention relates to a cell stably expressing a DNA construct comprising
(i) the adiponectin gene promoter region or a functionally equivalent variant thereof and
(ii) a reporter gene operatively coupled to said promoter region.

Finally, the invention relates to the use of a cell of the invention for identifying suitable compounds for the treatment or the prevention of metabolic diseases presenting altered adiponectin levels or in which it is necessary to alter the adiponectin levels.

### BRIEF DESCRIPTION OF THE DRAWINGS

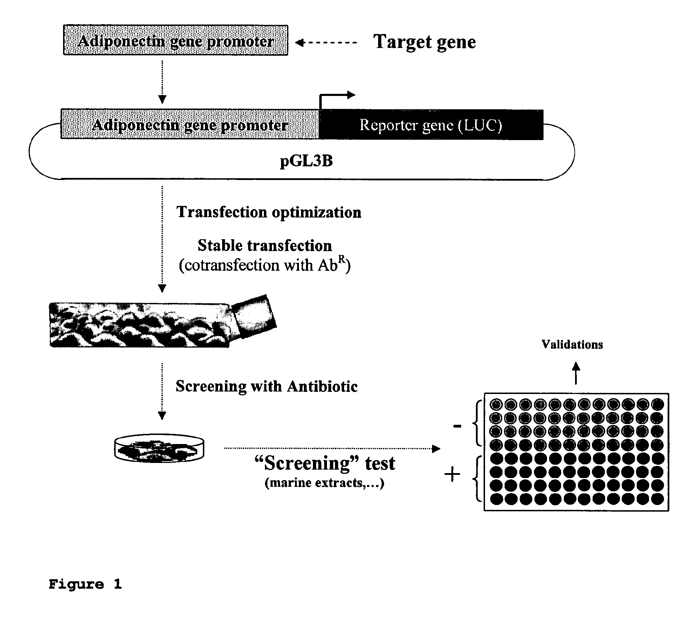

Figure 1: Experimental design of the method of the invention.

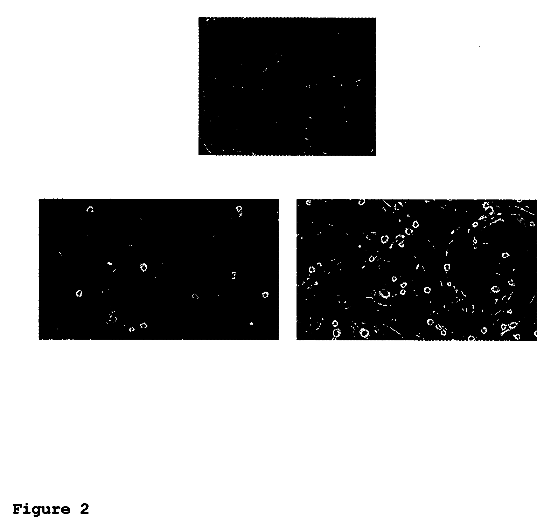

Figure 2: Morphology of the C2C12 cell line.

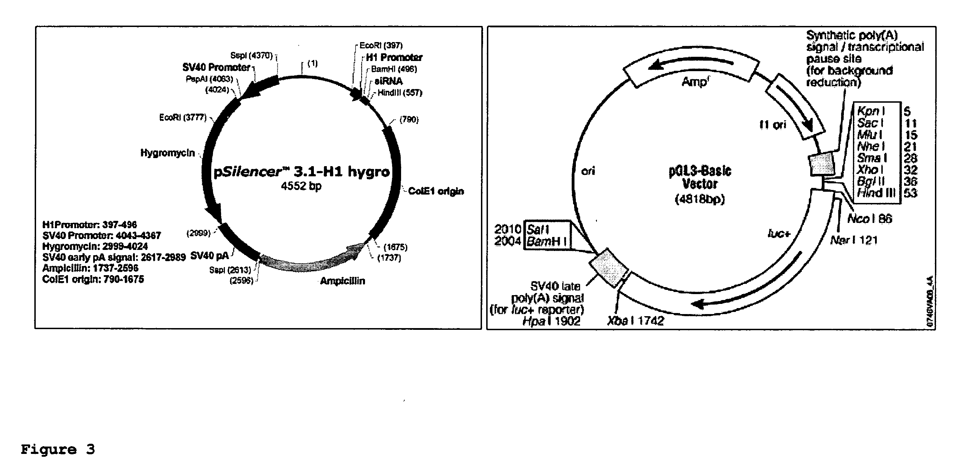

Figure 3: Diagram of the vectors used for cloning the DNA construct containing the target promoter and the reporter gene (pGL3B) and of the vector containing the hygromycin resistance gene (pSilencer 3.1-H1 Hygro).

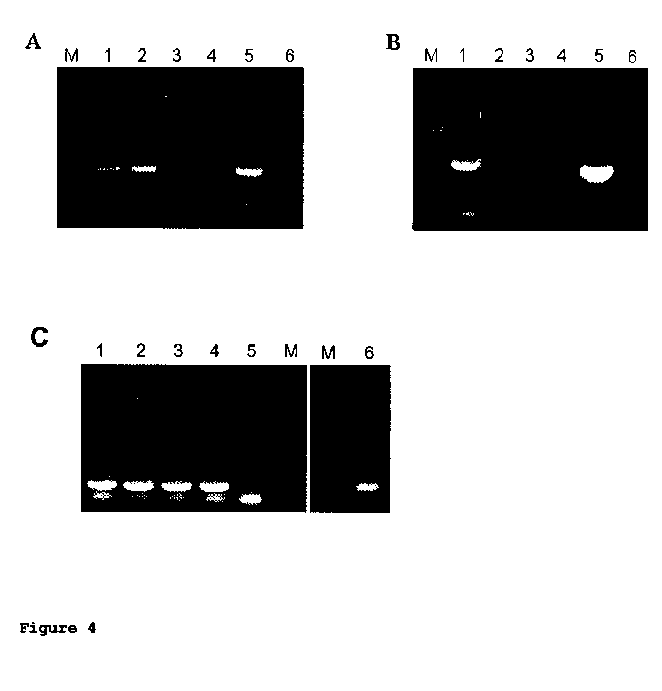

Figure 4: Detection of the RNA of the genes integrated in the stable C2C12 lines. Part A shows the RT-PCRs of the luciferase gene: the lanes with the letter M show the molecular weight markers; lane 1 shows the stable lines for the Acrp30 promoter; lanes 2 and 3 shows C2C12 cells transiently transfected with the pGL3C and pGL3B vectors, respectively, as positive RT controls; lanes 4, 5 and 6 show non-transfected C2C12 cells (as negative RT control), pGL3B plasmid (as positive RT-PCR control) and water (as negative RT-PCR control), respectively. Part B shows the RT-PCRs of the hygromycin resistance gene: the loading order is the same as that mentioned in part A, but the positive RT-PCR control (lane 6) is the p3.1-H1 plasmid; the molecular weight reporter is different to that used in part A. Part C shows the RT-PCRs of the β-action gene, as the endogenous gene used as a charge control in both RT-PCRs: the order of the samples is the same as that mentioned in parts A and B.

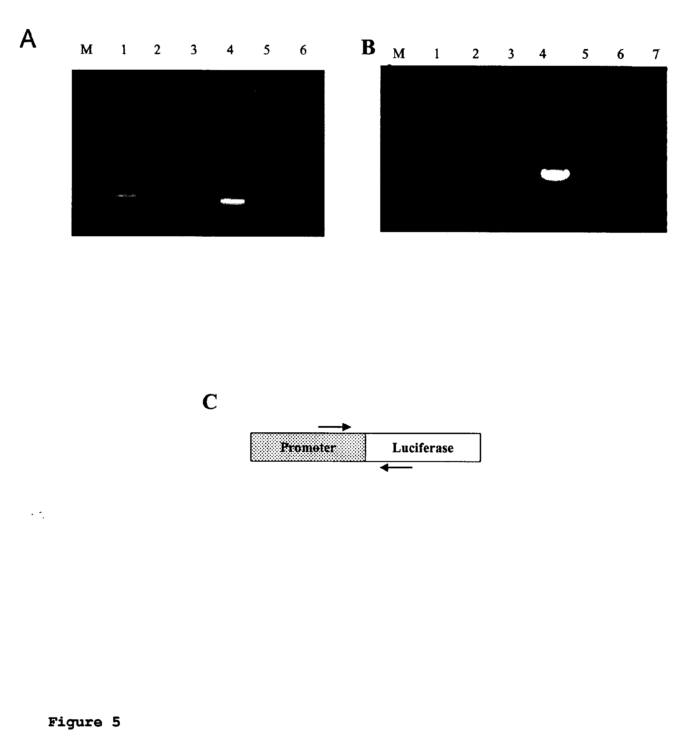

Figure 5: Detection of the DNA of the genes integrated in the stable lines. Part A shows the PCRs of the luciferase gene: the lanes with letter M show the molecular weight reporter; lanes 1 and 6 show the stable lines for the Acrp30 promoters and the stable line for the pGL3B vector, respectively; lanes 2, 3 and 5 show non-transfected Hek 293 and C2C12 cells, and water, respectively, as negative reaction controls; lane 4 shows the pGL3B plasmid as the positive reaction control. Part B shows the PCRs amplifying part of the Acrp30 promoter followed by part of the luciferase gene (as indicated in the diagram of part D): lane 1 shows the stable line for the Acrp30 promoter; lanes 2, 3 and 4 respectively show commercial genomic DNA and non-transfected Hek 293 and C2C12 cells, as negative reaction controls; lane 5 shows the plasmid with which the stable transfection was carried out on the cell line, as a positive reaction control; finally lanes 6, 7 and 8 show, as additional negative controls, the pGL3B plasmid, water and the stable cell line for the pGL3B vector, respectively.

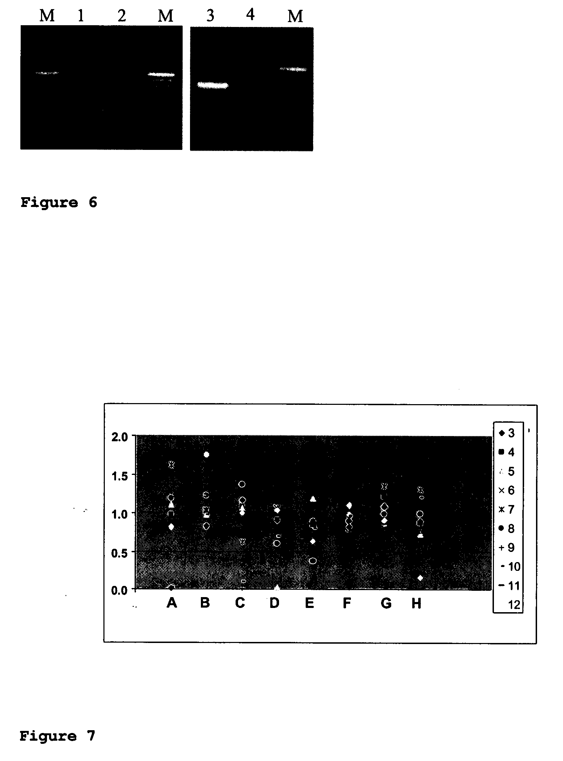

Figure 6: PCR for the detection of mycoplasma. Lanes 1 and 2 show the stable cell lines for the AdR1 promoter and the stable line for the pGL3B vector, respectively; lanes 3 and 4 show the positive and negative PCR controls, respectively, for detecting the presence/absence of mycoplasma. The lanes with letter M show the molecular weight reporter.
Figure 7: Luciferase activity of different marine extracts wherein the y-axis shows the ratio of luminescence obtained in the presence of each of the extracts in relation to the luminescence obtained in baseline conditions. The x-axis shows the values obtained in each of the 8 columns (samples A to H) and the values of the samples of each row (samples 3 to 12) are collected for each column.

### DETAILED DESCRIPTION OF THE INVENTION

The authors of the present invention have shown that, surprisingly, when cell lines are stably transfected with DNA constructs comprising the adiponectin gene promoter operatively coupled to a reporter gene, said cell lines are particularly useful for their use in highly sensitive screening methods for identifying compounds activating the transcription of the target gene and which are therefore useful for treating metabolic diseases associated to a deficient adiponectin expression. Thus, in a first aspect, the invention relates to a method (hereinafter, the method of the invention) for identifying suitable compounds for the treatment or the prevention of a metabolic disease presenting altered adiponectin levels or in which it is necessary to alter the adiponectin levels, comprising the steps of
(a)putting a cell in contact with a candidate compound in any degree of purity wherein said cell stably expresses a DNA construct comprising
   (i) the adiponectin gene promoter region or a functionally equivalent variant thereof and
   (ii) a reporter gene operatively coupled to said promoter region
   and
(b) detecting the reporter gene expression
wherein the assayed compound is identified as suitable for the treatment or prevention of said metabolic disease if it causes a variation in the reporter gene expression in an opposite direction to the existing alteration of the adiponectin levels in the metabolic disease compared to the alteration of the expression in the absence of said compound, or if it causes an alteration in the reporter gene expression in the same direction as the alteration to be achieved in the adiponectin levels.

Figure 1 shows a diagram of the method for identifying compounds according to the present invention.

In the context of the present invention, "metabolic disease" is understood as a disease selected from the group of type 2 diabetes mellitus, obesity, metabolic syndrome, arteriosclerotic cardiovascular disease, particularly atherosclerosis, dyslipidemia and lipodystrophy.

"Altered adiponectin levels" is understood as any disease in which there is an alteration in the expression of the adiponectin gene or of the protein encoded by said gene in cell lines or tissue derived from patients affected by said disease. The person skilled in the art will understand that there are conventional techniques for determining the expression levels of a certain gene in a certain cell or biological sample such as RT-PCR, Northern blot and the like to determine mRNA expression or different types of immunoassays (Western blot, ELISA, RIA, immunoprecipitation and the like) to determine the protein expression. It is thus possible to identify those pathological situations in which there is an alteration in the adiponectin levels. In a preferred embodiment, the alteration in adiponectin gene expression is a decrease and subsequently, the change in the reporter gene expression indicating that the candidate compound can be used for the treatment or prevention of said disease is an expression increase.

In a first step, the method of the invention involves putting a cell in contact with a candidate compound in any degree of purity. In the context of the present invention, a "cell" is understood as any cell in which the adiponectin gene promoter can promote the expression of a gene operatively coupled to said promoter. The expression of a gene through a promoter generally requires the presence of transcription factors which can recognize binding sequences in said promoter. The activation of the adiponectin gene promoter therefore requires the binding of a heterodimer formed by PPARγ and RXRα and is increased in the presence of the C/EBP-beta transcription factor. The cells which can be object of the present invention thus include all those cells expressing the necessary transcription factors for the transcriptional activation of the promoter being studied. Although it is possible to reconstitute the expression of said reporters in cells with very different origins, the use of cells expressing said transcription factors in an endogenous and constitutive manner is preferable. Thus, in a preferred embodiment, the cells being studied include higher eukaryotic cells, preferably mammal cells. The cells used in the present invention are preferably those in which the genes the promoters of which are being studied are expressed constitutively. The use of myoblasts or adipocyte precursor cells, such as for example the C2C12 myoblast cell line or the 3T3-L1 cell line, is therefore preferable for studying the adiponectin promoter. Alternatively, conventional cell lines can also be used, either directly if it is proved that they express sufficient amounts of suitable transcription factors or after the prior transfection of DNA constructs allowing the expression of said transcription factors. Suitable cells for this purpose include cells from the CHO, VERO, BHK, HeLa, COS, MDCK 293, 3T3, WI38 lines and the like. In a preferred embodiment, the cell used in the method of the invention is a C2C12 cell, corresponding to mouse muscle myoblasts.

Once the cell in which the DNA construct will be expressed has been screened, it is necessary to stably incorporate in said cell the DNA construct comprising the gene promoter being studied operatively coupled to a reporter gene. In the context of the present invention, "promoter" is understood as a DNA region to which RNA polymerase is bound and which therefore allows the transcription of genes which are operatively associated thereto. The present invention contemplates the use of the adiponectin gene promoter as well as of a functionally equivalent variant thereof. A "functionally equivalent variant" is understood as all those sequences derived from the SEQ ID NO:1 sequence by means of modification, insertion and/or deletion of one or more nucleotides, provided that the capacity of the sequence to promote the transcription of the genes operatively coupled to said sequences remains substantially intact. Persons skilled in the art will observe that sequences that are functionally equivalent to the previously indicated promoter regions can be obtained provided that the essential regions within said sequences in charge of the binding of the transcription factors remain intact. Functionally active variants of the adiponectin gene promoter must thus contain at least one region selected from the RXRα, PPARγ dimer binding region, the adiponectin transcription factor binding region ADD1/SREBP1c and the beta protein binding region binding to the CCAAT (C/EBP-2) potentiator. Persons skilled in the art will observe that the binding sites in the promoter regions object of the invention include those which can be determined by means of comparing with transcription factor binding sites bases such as TFSEARCH (Heinemeyer,T. et al., 1998, Nucleic Acid Res., 26:364-370). In a preferred embodiment, the adiponectin gene promoter comprises the sequence defined in SEQ ID NO:1.

The adiponectin promoter or the functionally equivalent variant thereof is operatively coupled to a reporter gene the expression of which can be easily detected. In the context of the present invention, reporter genes include luciferase, green fluorescent protein and variants thereof emitting fluorescence at different wavelengths (for example, DS-Red or red fluorescent protein), chloramphenicol acetyltransferase, β-galactosidase, alkaline phosphatase and radish peroxidase.

Once the chimeric gene comprising the adiponectin promoter and the reporter gene has been constructed, it must be introduced in a host cell. The DNA construct is introduced in the cells being studied using any of the transfection methods known by the persons skilled in the art (see sections 9.1 to 9.5 in Ausubel, F.M. et al., Current Protocols in Molecular Biology, John Wiley & Sons Inc; ringbou edition, 2003). The cells can particularly be transfected by means of precipitating DNA with calcium phosphate, DEAE-dextran, polybrene, electroporation, microinjection, liposome-mediated fusion, lipofection, infection by retrovirus and biolistic transfection.

For the purpose of obtaining stable expression of the DNA construct object of the invention, it is necessary to include in the transfection a gene encoding resistance to a certain antibiotic, such that those cell lines which have incorporated the DNA in the genome of those cell lines in which the DNA is in an extrachromosomal position can be screened. The gene which allows screening the cells can be provided forming part of the same vector containing the construct object of the invention or, alternatively, it can be provided separately by means of cotransfecting a second plasmid containing said resistance gene. In the latter case, the plasmid containing the DNA construct is provided to the transfection mixture in a molar excess with respect to the resistance gene, such that for each resistance gene integration event there is a high probability of integration of the gene containing the promoter being studied. The plasmid containing the DNA construct is preferably provided in an excess of at least 5 times with respect to the vector containing the resistance reporter.

Suitable resistance markers for screening cell lines which have integrated the construct in the genome include positive selection markers such as, for example, the neomycin resistance gene, conferring resistance to the aminoglycoside G418, the hygromycin phosphotransferase gene conferring resistance to hygromycin, the ODC gene conferring resistance to the ornithine decarboxylase inhibitor (2-(difluoromethyl)-DL-ornithine (DFMO)), the dihydrofolate reductase gene conferring resistance to methotrexate, the puromycin-N-acetyl transferase gene conferring resistance to puromycin, the ble gene conferring resistance to zeocin, the adenosine deaminase gene conferring resistance to 9-beta-D-xylofuranosyl adenine, the cytosine deaminase gene allowing the cells to grow in the presence of N-(phosphonacetyl)-L-aspartate, thymidine kinase allowing the cells to grow in the presence of aminopterine, the Xanthine-guanine phosphoribosyltransferase gene allowing the cells to grow in the presence of xanthine and in the absence of guanine, the E.coli trpB gene allowing the cells to grow in the presence of indole instead of tryptophan, the E.coli hisD gene allowing the cells to use histidinol instead of histidine. The selection gene is incorporated in a plasmid which can additionally include a suitable promoter for the expression of said gene in eukaryotic cells, (for example the CMV or SV40 promoters), an optimized translation initiation site (for example a site following the so-called Kozak rules or an IRES), a polyadenylation site such as for example the SV40 or phosphoglycerate kinase polyadenylation site, introns such as for example the beta-globulin gene intron.

The screening process of cells containing the DNA construct of interest stably integrated in the genome is carried out by means of a conventional screening process (see for example Ausubel, F.M. et al., Current Protocols in Molecular Biology (1997) 9.5.1-9.5.19). To that end, the cells are transfected with a vector or mixture of vectors and, after a recovery period, they are allowed to grow in a selective medium (either a medium containing the antibiotic against which the reporter confers resistance or a minimum medium containing the antimetabolite against which the reporter confers resistance). The cell colonies growing in selective medium are isolated and allowed to grow again in selective medium. Once cells have been obtained which can grow during repeated proliferation cycles in the presence of the selection marker, it may be convenient to eliminate said marker from the cells, particularly if the cells are to be transfected with another selection marker. To that end, recombinases may be used, particularly the Cre/Lox system. It is alternatively possible to amplify the number of copies of the selection marker, which results in a simultaneous amplification of the number of copies of the gene of interest, with the subsequent increase in its expression. To that end, the cells are grown in the presence of progressively greater concentrations of selection agent, which results in a screening of the cells which have undergone an amplification of the genes conferring resistance to such agent and normally of the adjacent or intermediate regions. DHFR is preferably used as a selection marker and the screening of cell lines in which there is an amplification of said gene is carried out in the presence of methotrexate.

A cell is normally considered to stably express a marker when the expression of said marker does not decrease with successive proliferation cycles, independently of the presence of selection agent in the culture medium.

Once a cell line which has stably integrated the DNA construct according to the invention in its genome is available, the cell is put into contact with a compound or preparation the effect of which on the transcription of the reporter gene is to be studied. According to the invention, "putting in contact" a cell with the candidate compound includes any possible way of taking the candidate compound inside the cell expressing the DNA construct. Thus, in the event that the candidate compound is a molecule with low molecular weight, it is enough to add said molecule to the culture medium. In the event that the candidate compound is a molecule with a high molecular weight (for example, biological polymers such as a nucleic acid or a protein), it is necessary to provide the means so that this molecule can access the cell interior. In the event that the candidate molecule is a nucleic acid, conventional transfection means can be used, as described previously for the introduction of the DNA construct. In the event that the candidate compound is a protein, the cell can be put in contact with the protein directly or with the nucleic acid encoding it coupled to elements allowing its transcription / translation once they are in the cell interior. To that end, any of the aforementioned methods can be used to allow its entrance in the cell interior. Alternatively, it is possible to put the cell in contact with a variant of the protein to be studied which has been modified with a peptide which can promote the translocation of the protein to the cell interior, such as the Tat peptide derived from the HIV-1 TAT protein, the third helix of the Antennapedia homeodomain protein from *D*.*melanogaster,* the VP22 protein of the herpes simplex virus and arginine oligomers (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

The compounds to be assayed can alternatively form part of an extract obtained from a natural source. The natural source can be an animal, plant source obtained from any environment, including but not limited to extracts of land, air, marine organisms and the like.

In a second step, the method of the invention includes determining the activity of the protein encoded by the reporter gene. At the same time as the activity of the reporter gene is determined in the presence of the compounds to be assayed, it is necessary to carry out parallel determinations of the baseline transcriptional activity only in the presence of the culture medium and/or of the carrier in which the compound to be assayed is dissolved or in which the extracts to be assayed have been prepared. Generally, those compounds or extracts with transcription promoting activity will be shown because they will give values greater than 1 for the ratio between the transcriptional activity in the presence of the compound or of the candidate extract and in the presence of carrier. The compounds or extracts in which the reporter gene activity ratio is at least 1.1, 1.2, 1.3, 1.4, 1.5, 1.6, 1.7, 1.8, 1.9 and 2 will preferably be considered positive.

In a preferred embodiment, the method of the invention for identifying suitable compounds for the treatment or the prevention of a metabolic disease in which said metabolic disease is characterized by a decrease in the expression of a gene in which the change of reporter gene expression is an increase of the expression.

The method for detecting the reporter gene expression involves putting the cells in contact with a compound which can generate a colored or fluorescent product in the presence of the product encoded by the reporter gene. Thus, if the enzyme is alkaline phosphatase, chromogenic substrates of the type of p-nitrophenyl phosphate (p-NPP), 5-bromo-4-chloro 3-indolyl phosphate/ nitroblue tetrazolium (BCIP/NBT), Fast-Red/naphthol-AS-TS phosphate or fluorogenic substrates of the type of 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), Fast Blue BB, Fast Red TR or diazonium salts of Fast Red Violet LB can be used.

If the reporter gene encodes a peroxidase, chromogenic substrates of the type of 2,2-azinobis (3-ethylbenzothiazoline-6-sulfonic acid) (ABTS), o-phenylenediamine (OPD), 3,3',5,5'-tetramethylbenzidine (TMB), o-dianisidine, 5-amino salicylic acid, 3-dimethylamino benzoic acid (DMAB) and 3-methyl-2-benzothiazoline hydrazone (MBTH), 3-amino-9-ethylcarbazole (AEC) and 3,3'-diaminobenzidine tetrahydrochoride (DAB) or fluorogenic substrates of the type of 4-hydroxy-3-methoxyphenylacetic acid, reduced phenoxazines and reduced benzothiazine, including the reagents Amplex^{®} Red and Amplex UltraRed and reduced dihydroxanthenes can be used.

If the reporter gene encodes a glucosidase, chromogenic substrates of the type of o-nitrophenyl-β-D-galactoside (o-NPG), p-nitrophenyl-β-D-galactoside and 4-methylumbelliferyl-β-D-galactoside (MUG) for β-D-galactosidase and fluorogenic substrates of the type of resorufin beta-D-galactopyranoside, fluorescein digalactoside (FDG), fluorescein diglucuronide, 4-methylumbelliferyl beta-D-galactopyranoside, carboxyumbelliferyl beta-D-galactopyranoside and coumarin beta-D-galactopyranoside.

In a preferred embodiment, the reporter gene encodes luciferase and the detection is carried out measuring the luminescence emitted by said enzyme in the presence of ATP. The luminescence is determined using commercial kits, such as for example, the enhanced luciferase assay kit (Analytical Luminescence Laboratory, MI). The method for identifying compounds of the invention is preferably carried out using high-throughput screening (HTS), such that a high number of samples can be assayed simultaneously. The HTS are preferably carried out in multi-well plates, preferably in 96-well plates, which facilitates the detection of luciferase activity in each sample given that there are illuminometers which can directly accept 96-well culture plates.

In the event that the candidate compound forms part of a more or less complex mixture, the invention additionally comprises one or several steps (iii) of fractioning said mixture and the repetition of steps (i), (ii) and (iii) of the method of the invention a variable number of times until the compound of the mixture responsible for the transcription promoting activity is isolated. Methods for fractioning the compounds present in a mixture include chromatography (thin layer, gas, gel molecular exclusion, affinity chromatography) crystallization, distillation, filtration, precipitation, sublimation, extraction, evaporation, centrifugation, mass spectroscopy, adsorption and the like.

In another aspect, the invention relates to host cells used in the method of the invention (hereinafter the cells of the invention) and is characterized by stably expressing a DNA construct comprising
1. the adiponectin gene promoter region or a functionally equivalent variant thereof and
2. a reporter gene operatively coupled to said promoter region.

The definitions of "promoter", "functionally equivalent variant of the promoter region", "stable expression", "altered expression", "metabolic disease", "reporter gene" have been explained in detail above while describing the method of the invention. The adiponectin gene promoter region preferably comprises the SEQ ID NO:1 sequence.

Suitable cells for carrying out the present invention are those cells in which, as defined above, the adiponectin gene promoter can promote the expression of a gene operatively coupled to said promoter. In a preferred embodiment, the cell used as a host cell for the method of the invention is a C2C12 cell.

The present invention also contemplates the use of the cells of the invention for identifying suitable compounds for the treatment and/or the prevention of a metabolic disease presenting altered adiponectin levels or in which it is necessary to alter the adiponectin levels. The metabolic diseases are preferably selected from the group of obesity, type 2 diabetes mellitus and atherosclerosis.

The present invention is additionally explained below by means of examples. It must by no means be interpreted as a limitation of the scope of the invention as it is defined in the claims.

### EXAMPLES

### EXAMPLE 1

### Materials

The cell line C2C12 corresponds to an isolated mouse muscle tissue *(Mus musculus)* line showing a myoblast morphology and an adherent phenotype (Figure 2).

The region corresponding to the human adiponectin gene promoter corresponds to 2274 base pairs before the transcription initiation site (SEQ ID NO:1).

### EXAMPLE 2

### Amplification and cloning of the human adiponectin gene promoter region

The sequence corresponding to the human adiponectin gene promoter region has been obtained from the UCSC Genome Bioinformatics (http://genome.ucsc.edu/) and Ensembl (http://www.ensembl.org/index.html) programs. The oligonucleotides used to amplify the promoter sequence were designed on said sequence covering a 2274 bp region from nucleotide -15 to the nucleotide -2288 (both inclusive). Both oligonucleotides include a TATA box at the start of the sequence. A recognition site for the Mlu I restriction enzyme was further included in the F (forward) olgonucleotide and a recognition site for Xho I was included in the R (reverse) enzyme, for the purpose of facilitating its subsequent cloning into the pGL3-Basic (Promega) vector. The sequences of said oligonucleotides are detailed below:
Adiponectin F: ACGCGTTAGTGGAGACGGGGTTTCAC (SEQ ID NO: 2)
Adiponectin R: CTCGAGCAGGCAGTCCCCACCTCAC (SEQ ID NO: 3)

Commercial human genomic DNA from two independent laboratories Roche and Novagen, was used for the amplification of the promoter region of said gene. The amplification was carried out using the GC-Rich PCR System kit (Roche), following the instructions provided by the manufacturer and with a program including an initial treatment of 3 minutes at 95°C, 10 cycles of 30 seconds at 95° C, 30 seconds at 58°C and 3 minutes at 68°C and 20 cycles of 30 seconds at 95°C, 30 seconds at 58°C, 3 minutes at 68°C and 5 minutes at 66°C and, finally a final elongation step of 7 minutes at 68°C.

### EXAMPLE 3

### Obtaining a cell line stably expressing a reporter gene under the control of the adiponectin promoter

The C2C12 cells were seeded in 6-well plates (160000 cells per well) in complete DMEM with 10% fetal bovine serum (FBS).

On the next day, the constructs which contained the pGL3B vector with the adiponectin gene promoter region together with the p*Silencer* 3.1-H1 vector, containing the hygromycin resistance gene (Figure 3), in a 2.5:0.5 µg ratio (Luciferase: hygromycin resistance) were cotransfected. To that end, culture medium (600 µl) was mixed with the previously indicated DNA amounts, they were incubated for 5 minutes at room temperature, the reagent TRANSFAST (Promega) (1 µg DNA per every 3 µl of Transfast) was added and the mixture was incubated for 15 minutes at room temperature. After this time, the medium was removed from the wells and the transfection volume was added dropwise to the cells. The mixture was then incubated for 1 hour at 37°C. The transfection mixture was then removed and complete medium was added. Parallel to the transfection of the constructs of interest, transfections were carried out using reporter genes of green fluorescent protein (GFP) and β-galactosidase in the same conditions as the constructs of interest, to thus estimate the efficiency of the transfection process, as described below.

On the next day, the transfection efficiency was determined by means of determining
a) the GFP gene expression, using a fluorescence microscope,
b) β-galactosidase gene expression, with a specific stain kit which allows observing the cells under the microscope once they have been stained

1/100 and 1/150 passages of the cells ("dilutions") were carried out, seeding them already on hygromycin in new P6 plates, starting from the wells in which a 40-60% transfection percentage was obtained. The amount of hygromycin added was 700 µg/ml (1.33 mM). A hygromycin resistance curve with different drug concentrations on cells seeded in P6 had previously been made to choose the suitable amount of drug to be used in the screening.

In the following days, the cells were always maintained with hygromycin and were observed under the microscope to see the evolution of the possible clones. The clones covering a ten-times magnification lens when observed under a microscope were considered large enough to be isolated from that plate and set up a stable cell line from it. To that end, the culture medium was removed, the cells are washed twice with PBS, cloning rings were placed (with petroleum jelly) on each clone and they were pressed with clamps so that they adhered through the petroleum jelly. 20-30 µl of trypsin were then added to each ring and incubated for 5 minutes at 37°C. Once it was verified that all the clone cells had been suitably removed from the plate, 100 µl of culture medium were added to stop the trypsinization reaction, it was pipetted up and down taking care to not move the ring and the volume of medium with hygromycin to reach 1 ml of medium in P24 was added. The cultures were successively escalated from P24 to P6 plates and from P6 to T25 plates. When the cells of the clone reached confluence in the T25 plate, a vial was frozen and the transcriptional activity of the promoter which it had stably integrated was measured through the luciferase reporter gene. The clones that were positive in the study were maintained in hygromycin and the luciferase activity was measured periodically in order to control that they do not lose said activity.

Once stable expression clones were obtained, they were maintained in DMEM with 10% fetal bovine serum (Clinus, Cultek) filtered and supplemented with 2 mM glutamine (Gibco), antibiotic-antifungal agent (Gibco; 350 U/ml penicillin G, 350 µg/ml streptomycin and 0.875 µg/ml Amphotericin B) and 700 µg/ml hygromycin B (Calbiochem).

For the subculture of the cell lines, the cells were prevented at all times from reaching confluence for which two 1/6 passages were carried out per week. To that end, the cultures were washed twice with PBS, 2ml of trypsin were added (75 cm² for a T75,) and the bottle was incubated for 3 minutes at 37°C, the trypsinization reaction was stopped by adding 4 ml of culture medium (described previously) and the total volume was centrifuged for 5 minutes at 100 x g (∼ 900 rpm). The cell precipitate was washed with 4 ml of PBS and it was centrifuged again in the same conditions. The precipitate was then resuspended in culture medium and the necessary volume was seeded to obtain the 1/6 passage in a new bottle.

### EXAMPLE 4

### Determination of the luciferase activity and of the presence of the integrated gene by means of RT-PCR

To determine the luciferase activity, between 50000 and 1200000 cells were seeded in each well and in a P24 plate in duplicate. After approximately 24 hours, the medium was removed from the cells and each well was washed with PBS 1x (because DMEM interferes in the measurement of the reporter gene activity). 100 µl of luciferase developing agent (Bright-GloTM Luciferase Assay system- Promega) were then added to each well. This reagent carries the reporter gene substrate and luminescence is generated as a product of the enzymatic reaction. The cells which had acquired the reporter gene, and therefore the adiponectin promoter, showed luminescence upon being in the presence of said substrate. To record the measurement of luciferase activity, the plate was shaken for 30 seconds right after adding the developing reagent and after 2 minutes, the volume was collected and transferred to a 96-well plate (P96), which was read in an illuminometer using the program with a duration of 5 seconds and the arbitrary luminescence units were recorded for each obtained clone or, in other words, for each stable cell line.

The correct integration of the genes was additionally to be verified in a more reliable manner, for which the RNA and DNA of the stable lines were analyzed. To that end, RNA was extracted from each of the cell lines and an RT-PCR was carried out with suitable primers to detect the presence of the mRNAs of luciferase and of the protein conferring hygromycin resistance in said lines (Figure 4). It could thus be verified that the cloned and integrated human promoting sequence carries out its transcriptional function correctly.

In addition, the genomic DNA extracted from each of the cell lines was used to carry out PCR and thus detect the presence of the reporter gene (luciferase) and of the hygromycin resistance gene in said lines (Figure 5A). In addition, a PCR was carried out using a forward oligonucleotide in the promoter sequence and a reverse oligonucleotide in the luciferase gene sequence, which allowed showing that in stable cell lines, the luciferase gene had been integrated under the control of the target promoter used for the PCR (Figure 5B). It can thus be assured that the luciferase enzyme activity reflected in the screening tests is only the result of the transcriptional activity carried out by the target promoter on said reporter gene and therefore, it has not been integrated with any other endogenous promoter in the cell.

The presence of mycoplasma in the culture of said stable lines was also tested regularly, said test being negative from the start (Figure 6).

### EXAMPLE 5

### Screening of marine extracts

Before any screening experiment, the suitable number of cells for carrying out such experiments must first be provided. To that end, 2 T75 plates were seeded with approximately 1x10⁶ cells in each bottle, such that after four days, the cells had reached confluence and were ready for the screening protocol detailed below to be carried out.

For the screening, 30000 cells per well were seeded in white P96 plates because these plates allow amplifying the luminescence signal and improving the reading. The cells were grown in 100 µl of hygromycin-free medium.

The cells were stimulated with the extracts 24 hours after the seeding in the following conditions:
- cells to which only complete medium had been added (baseline transcription),
- cells to which medium had been added in which the extracts to be assayed are dissolved (vehicle),
- cells to which a known commercial activator of the target promoter had been added, thus having a positive control of the transcriptional activation and of the screening protocol and which are useful as a reference when screening those positive extracts increasing the activity of the promoters to be studied, because they provide an activation ratio which is calculated as follows: by dividing the average luminescence signal increase in the presence of the commercial activator by the average luminescence signal provided by the baseline activity of the target promoter in the presence of the vehicle. In the case of the adiponectin promoter, rosiglitazone (RGZ), a known adiponectin gene transcription activator, was initially chosen as the commercial activator. This compound showed a maximum activity at 20 µM. RGZ was subsequently substituted with genistein and daidzein, given that both compounds are also described in the literature as possible PPARγ activators, they also show a maximum concentration of 20 µM as the concentration at which the greatest transactivation of the target promoter was obtained. Daidzein was chosen because it was less toxic for the cells than Genistein and because it was more potent than RGZ in the screening assays.
- Cells to which 25 µg/ml of marine extract to be assayed had been added. To that end, an extract amount of 2.5 mg/ml ("parent" plate) was diluted into another plate ("daughter" plate) to a concentration of 200 µg/ml. The final volume in each well was 150 µl between the culture medium and the crude extract volume.

The luciferase activity directed by the target promoters was developed 48 hours after the stimulation with the extracts, according to the protocol described in example 4, but modified such that once the medium was removed from the cells and they were washed with PBS 1x, 40 µl of luciferase developing reagent were added to each well and the plate was read after 2 minutes.

Luminescence values measured in RLUs (Relative Luciferase Units) obtained in the presence of each of the marine extracts which have been assayed were then plotted against the average luminescence signal obtained in baseline conditions (in the presence of the vehicle). Any assay in which an activation ratio ≥2 is obtained when the cells are treated with the commercial activator is considered valid. In these cases, only the ratios obtained when the cells are treated with the 80 marine extracts in each plate (columns 3 to 12) were plotted, resulting in a graph similar to that shown in Figure 7.

The extracts obtaining in the assay an activation ratio ≥1.5 in the case of extracts from macroorganisms and an activation ratio ≥2 in the case of extracts from microorganisms were considered positive. These cut-off values were chosen based on the analysis of the results obtained until reaching the first positive extract for the primary screening assay.

From the extracts which were positive in the screening, a second screening round was carried out in which each of the extracts at concentrations of 5, 15 and 25 µg/ml were assayed in duplicate. The assay was carried out in conditions identical to those described for the primary screening. Whenever possible, and depending on the sample availability, the second round was carried out by assaying in parallel the "parent" plate and the "daughter" plate, with which the primary screening was carried out. The second round was always carried out in duplicate, using the following samples
- baseline transcription
- transcription in the presence of commercial activators
- transcription in the presence of positive extract at 5 mg/ml
- transcription in the presence of positive extract at 10 mg/ml
- transcription in the presence of positive extract at 15 mg/ml

Once the results have been processed, all the values referring to the average baseline transcriptional activity, those that were positive (according to the aforementioned ratios) were screened for their subsequent chemical fractionation.

The fractionation samples of each screened extract were assayed reproducing the scheme used in the second screening round of the crude extracts, but modifying the range of concentrations at 1, 5 and 10 µg/ml. The assay of the samples obtained from the fractionation of each positive extract is thus continued until the structure of the active ingredient responsible for the transactivation observed on the promoter in question is deduced. Regardless of the cell line in which transactivation was initially obtained (in the primary screening), the samples of compounds obtained from fractionation are assayed in all the cell lines available at that time.

Once the structure of a pure compound has been obtained, a dose-response curve (µM) is made to know: a) the concentration at which the maximum transactivation of the promoter is obtained in the presence of that compound and b) the concentration at which 50% transactivation, over the maximum observed (EC₅₀), is obtained.

### EXAMPLE 6

### Screening of compounds obtained by chemical synthesis

Before any screening experiment, the suitable number of cells for carrying out such experiments must first be provided. To that end, 2 T75 plates were seeded with approximately 1x10⁶ cells in each bottle, such that after four days, the cells had reached confluence and were ready for the screening protocol detailed below to be carried out.

For the screening, 30000 cells per well were seeded in white P96 plates because these plates allow amplifying the luminescence signal and improving the reading. The cells were grown in 100 µl of hygromycin-free medium.

The cells were stimulated with the extracts 24 hours after the seeding in the following conditions:
- cells to which only complete medium had been added (baseline transcription),
- cells to which medium had been added in which the extracts to be assayed are dissolved (vehicle),
- cells to which a known commercial activator of the target promoter had been added, thus having a positive control of the transcriptional activation and of the screening protocol and which are useful as a reference when screening those positive extracts increasing the activity of the promoters to be studied, because they provide an activation ratio which is calculated as follows: by dividing the average luminescence signal increase in the presence of the commercial activator by the average luminescence signal provided by the baseline activity of the target promoter in the presence of the vehicle. In the case of the adiponectin promoter, rosiglitazone (RGZ), a known adiponectin gene transcription activator, was initially chosen as the commercial activator. This compound showed a maximum activity at 20 µM. RGZ was subsequently substituted with genistein and daidzein, given that both compounds are also described in the literature as possible PPARγ activators, they also show a maximum concentration of 20 µM as the concentration at which the greatest transactivation of the target promoter was obtained. Daidzein was chosen because it was less toxic for the cells than Genistein and because it was more potent than RGZ in the screening assays.
- Cells to which 25 µg/ml of the compound to be assayed had been added. To that end, an extract amount of 2.5 mg/ml ("parent" plate) was diluted into another plate ("daughter" plate) to a concentration of 200 µg/ml. The final volume in each well was 150 µl between the culture medium and the compound volume dissolved in its vehicle.

The luciferase activity directed by the target promoters was developed 48 hours after the stimulation with the compounds, according to the protocol described in example 4, but modified such that once the medium was removed from the cells and they were washed with PBS 1x, 40 µl of luciferase developing reagent were added to each well and the plate was read after 2 minutes.

Luminescence values measured in RLUs (Relative Luciferase Units) obtained in the presence of each of the compounds which have been assayed were then plotted against the average luminescence signal obtained in baseline conditions (in the presence of the vehicle). Any assay in which an activation ratio ≥2 is obtained when the cells are treated with the commercial activator is considered valid. In these cases, only the ratios obtained when the cells are treated with the 80 compounds in each plate (columns 3 to 12) were plotted, resulting in a graph similar to that shown in Figure 7.

The compounds obtaining an activation ratio ≥2 in the assay were considered positive.

From the compounds which were positive in the screening, a second screening round was carried out in which each of the compounds at concentrations of 5, 15 and 25 µg/ml were assayed in duplicate. The assay was carried out in conditions identical to those described for the primary screening. Whenever possible, and depending on the sample availability, the second round was carried out by assaying in parallel the "parent" plate and the "daughter" plate, with which the primary screening was carried out. The second round was always carried out in duplicate, using the following samples
- baseline transcription
- transcription in the presence of commercial activators
- transcription in the presence of positive compound at 5 mg/ml
- transcription in the presence of positive compound at 10 mg/ml
- transcription in the presence of positive compound at 15 mg/ml

Once the results have been processed, all the values referring to the average baseline transcriptional activity, those that were positive (according to the aforementioned ratios) were screened.

A dose-response curve (µM) is made for the positive compounds to know: a) the concentration at which the maximum transactivation of the promoter is obtained in the presence of that compound and b) the concentration at which 50% transactivation, over the maximum observed (EC₅₀), is obtained.

## Claims

1. A method for identifying suitable compounds for the treatment or the prevention of a metabolic disease presenting altered adiponectin levels or in which it is necessary to alter the adiponectin levels, comprising the steps of
(a)putting a cell in contact with a candidate compound in any degree of purity wherein said cell stably expresses a DNA construct comprising
(i) the adiponectin gene promoter region or a functionally equivalent variant thereof and
(ii) a reporter gene operatively coupled to said promoter region
and
(b)detecting the reporter gene expression
wherein the assayed compound is identified as suitable for the treatment or prevention of said metabolic disease if it causes a variation in the reporter gene expression in an opposite direction to the existing alteration of the adiponectin levels in the metabolic disease compared to the alteration of the expression in the absence of said compound, or if it causes an alteration in the reporter gene expression in the same direction as the alteration to be achieved in the adiponectin levels.

2. A method according to claim 1, wherein the metabolic disease presenting a decrease in the adiponectin expression levels and wherein the change of reporter gene expression detected in step b) is an increase of expression.

3. A method according to claim 2, wherein the metabolic disease is selected from the group of obesity, type 2 diabetes mellitus and atherosclerosis.

4. A method according to claim 3, wherein the adiponectin gene promoter region comprises the SEQ ID NO:1 sequence.

5. A Un method according to any of claims 1 to 4, wherein the cell is a C2C12 cell.

6. A method according to any of claims 1 to 5, wherein if the candidate compound forms part of a mixture, the method additionally comprises one or several steps (c) of fractioning the assayed mixture and repeating steps (a), (b) and (c) with each of the obtained fractions until the compound in the mixture which is suitable for the treatment or the prevention of metabolic diseases is isolated.

7. A cell stably expressing a DNA construct comprising
(i) the adiponectin gene promoter region or a functionally equivalent variant thereof and
(ii) a reporter gene operatively coupled to said promoter region.

8. A cell according to claim 7, wherein the adiponectin gene promoter region comprises the SEQ ID NO:1 sequence.

9. A cell according to claims 7 or 9, wherein the cell is a C2C12 cell.

10. The use of a cell according to any of claims 7 to 9 for identifying suitable compounds for the treatment or the prevention of a metabolic disease presenting altered adiponectin levels or in which it is necessary to alter the adiponectin levels.

11. The use according to claim 10, wherein the metabolic disease is selected from the group of obesity, type 2 diabetes mellitus and atherosclerosis.
